# EUROPEAN PATENT SPECIFICATION

(11) **EP 3 860 620 B1**
(45) Date of publication and mention of the grant of the patent: **06.12.2023**
(21) Application number: 19795326.8
(22) Date of filing: 30.09.2019
(51) Int. Cl.: A61K 36/28, A61K 33/00, A61P 35/00, A61K 9/00, G01N 33/574

(54) **FORMULATION COMPRISING OZONISED OIL IN THE TREATMENT OF A TUMOUR**
FORMULIERUNG ENTHALTEND EIN OZONIERTES ÖL ZUR TUMORBEHANDLUNG
FORMULATION COMPRENANT UNE HUILE OZONIZÉE POUR TRAITER UNE TUMEUR

(30) Priority: 01.10.2018 IT 201800009063
(43) Date of publication of application: 11.08.2021
(73) Proprietor: O3 ZONE LIMITED, London N11 3NE (GB)
(72) Inventor: CAROCCI, Giancarlo, 41016 Sevilla (ES)
(74) Representative: Casci, Tamara
(86) International application number: PCT/IB2019/058298
(87) International publication number: WO 2020/070623

(56) References cited:
- WO-A2-2008/050157
- US-A1- 2006 074 129
- DATABASE WPI Week 201727 Thomson Scientific, London, GB; AN 2017-08542Q XP002792134, & CN 106 265 722 A (HOU J) 4 January 2017 (2017-01-04)

## Description

### Field of application

The present invention relates to the use of a formulation comprising ozonized oil in the prevention and/or treatment of a tumour, in particular a malignant tumour (cancer).

### Prior art

The neoplastic cell differs from the normal one in many aspects including the block of the mitochondrial function, aimed at inhibiting apoptosis (spontaneous cell death), which constitutes a fundamental impediment for the development of cancer. The mitochondrial block that occurs in the neoplastic cell is known as the Warburg effect.

Recent experimental research shows that neoplastic cells are favoured, in their growth, by anti-oxidant molecules but are contrasted by pro-oxidant situations. In particular, the cancer stem cells, which give rise to resistance to chemo/radiotherapy and relapses, are characterized by a reducing environment and are therefore very sensitive to the cytotoxic effects of the oxidative damage (Zhou et al., Reactive oxygen species in normal and tumour stem cells. Adv Cancer Res. 2014;122:1-67). The mitochondrion is the main endogenous source of oxidizing molecules.

Today, the increase in oxidative damage represents therefore a strategy for cancer therapy (Liu et al., 2015, Increased Oxidative Stress as a Selective Anticancer Therapy. Oxid Med Cell Longev. Article ID294303).

Some attempts to use ozone as a source of oxidizing species in cancer therapy have been made. In particular ozone as a gas (Rossmann et al., Intraperitoneal oxidative stress in rabbits with papillomavirus-associated head and neck cancer induces tumoricidal immune response that is adoptively transferable. Clin Cancer Res. 2014; 20 (16) :4289-4301)) or as an ozonized aqueous solution by injection was used (Kuroda et al., The Safety and Anti-Tumor Effects of Ozonated Water in Vivo. Int J Mol Sci. 2015; 16(10) :25108-25120)). These studies have shown specific cytotoxic effects on the neoplastic tissue in the absence of damage to healthy tissues. However, the therapeutic effect obtained was significant but transient.

The need is therefore felt in the sector to provide a formulation based on ozonizing species for the prevention and/or treatment of a tumour, in particular a malignant tumour (cancer), which overcomes the drawbacks and limits of the formulations of the prior art.

The technical problem underlying the present invention is therefore that of providing a formulation comprising ozonizing species that is effective in the prevention and/or treatment of a tumour, in particular a malignant tumour (cancer), which has a prolonged therapeutic effect over time.

The malignant tumour can be a solid tumour, for example a prostate, liver, lungs, breast, colorectal, pancreas or encephalon tumour; a cutaneous (skin) tumour; a mucosal tumour, for example of the mouth, nose, anus, vulva or vagina; or a liquid tumour, such as a hematopoietic tumour.

In particular, the technical problem underlying the present invention is that of providing such a formulation that has a therapeutic effect for a time of at least six months from the end of the therapy, preferably of at least 5 years.

A further technical problem underlying the present invention is that of providing such a formulation that is effective in preventing the formation of metastases.

A further technical problem underlying the present invention is that of providing such a formulation that has a higher content of ozonides than the oxidizing formulations of the prior art described above (30 meq O₂/kg), in particular greater than 100 meq O₂/kg, still more particularly greater than 500 meq O₂/kg.

A further technical problem underlying the present invention is that of providing such a formulation that has high tolerability, that is a lower number and intensity of undesired side effects with respect to the therapies currently used.

In particular, the formulation should be able to exert an anti-cancer effect without damaging the non-neoplastic cells, that is the healthy ones.

A further technical problem underlying the present invention is that of providing such a formulation that allows an easy personalisation of the dose.

### Summary of the invention

A similar problem has been solved according to the invention by a formulation comprising at least one ozonized oil for use in the prevention and/or treatment of a tumour, preferably a malignant tumour.

Preferably, such formulation is for use by administration chosen from oral, topical, by injection (for example by infiltration), by inhalation (for example in the form of an aerosol), and combinations thereof.

Preferably, such formulation is for use by topical administration and/or administration by injection (for example by infiltration).

The ozonized oil has a content of ozonides from 500 to 1500 meq O₂/kg, more preferably from 600 to 1400 meq O₂/kg, even more preferably from 700 to 1300 meq O₂/kg.

In a preferred embodiment, the ozonized oil has a content of ozonides greater than 800 and lower than 1500 meq O₂/kg, more preferably greater than 900 and lower than 1400 meq O₂/kg, even more preferably greater than 1000 and lower than 1300 meq O₂/kg, the most preferably greater than 1100 and lower than 1200 meq O₂/kg.

The content of ozonides can be measured according to techniques known in the field, for example by the method for determining the number of peroxides described in the Regulation (EEC) No. 2568/91 of 11 July 1991 concerning the characteristics of olive oils and olive-residue oils and the relevant methods of analysis, Annex III.

Preferably, the ozonized oil is a vegetable oil that has undergone an ozonizing process, more preferably chosen from: sunflower oil, olive oil, peanut oil, argan oil, grapeseed oil, jojoba oil, soybean oil, corn oil, palm oil, cottonseed oil, rapeseed oil, coconut oil, castor oil, linseed oil, borage oil, evening primrose oil, and mixtures thereof.

According to a particularly preferred embodiment, the ozonized oil is sunflower seed oil (hereinafter also "sunflower oil"), peanut seed oil (hereinafter also "peanut oil"), or mixtures thereof. In fact, it is preferable to use an oil substantially free of any intrinsic anti-oxidizing activity.

Preferably, the ozonized oil is substantially free of antioxidant agents.

According to a preferred embodiment, the ozonized oil is chosen from: sunflower oil, peanut oil, argan oil, grapeseed oil, jojoba oil, soybean oil, corn oil, palm oil, cottonseed oil, rapeseed oil, coconut oil, castor oil, linseed oil, borage oil, evening primrose oil, and mixtures thereof, more preferably sunflower oil and/or peanut oil.

Preferably, the ozonized oil is obtained through an ozonisation process which uses highly purified gaseous oxygen for the introduction of oxygen.

Preferably, the formulation is to be administered to an animal or human mammal suffering from a tumour, more preferably a malignant tumour.

Preferably, the tumour is chosen from a solid tumour, for example a prostate, liver, lungs, breast, colorectal, pancreas, or encephalon tumour; a cutaneous (skin) tumour; a mucosal tumour, for example of the mouth, nose, anus, vulva or vagina; and a liquid tumour, such as a hematopoietic tumour.

Preferably, the aforesaid formulation acts on the neoplastic cell by oxidation of the mitochondrial membranes.

According to a preferred embodiment, the aforesaid formulation is for use by topical administration.

The formulation for topical use object of the present invention can consist of the aforesaid at least one ozonized oil or it can comprise the aforesaid at least one ozonized oil, preferably in a concentration, in weight percent with respect to the total weight of the formulation, from 1 to 50%, even more preferably from 2 to 20%, the most preferably from 3 to 15%.

In a preferred embodiment, the formulation for topical use of the present invention further comprises a pharmaceutically acceptable vehicle, compatible with the ozonized oil.

Preferably, the formulation for topical use of the invention is in a form chosen from oil, gel, and emulsion, more preferably oil.

The gel can be a hydrogel or a lipogel.

Preferably, the hydrogel comprises, in weight percent on the total weight of the hydrogel, from 3 to 30%, more preferably from 5 to 20%, the most preferably from 8 to 15% of at least one ozonized oil.

The term "hydrogel" here means that the formulation is in the form of a gelled hydrophilic solution in which the ozonized oil is dispersed in an aqueous medium.

The term "lipogel" here means that the formulation is in the form of a gelled lipophilic solution in which the ozonized oil is dispersed in a lipidic medium.

According to a preferred aspect of the present invention, the topical use for the prevention and/or treatment of a tumour is performed by topical application of the formulation on the affected tissue, preferably for a period of more than three days and less than sixty days, more preferably from ten to thirty days.

Preferably, the topical application of the formulation is performed once or twice a day, more preferably twice a day.

Preferably, the topical application is performed by applying an amount of the formulation on the affected tissue that is sufficient to cover the neoplastic lesion, more preferably sufficient to cover the neoplastic lesion and at least part of the perilesional skin.

Perilesional skin is defined as the cutaneous surface that extends from the edge of the lesion up to one centimetre from it.

According to a preferred aspect of the present invention, the formulation for topical use comprises an ozonized oil which has a content of ozonides greater than 800 and lower than 1500 meq O₂/kg, more preferably greater than 900 and lower than 1400 meq O₂/kg, even more preferably greater than 1000 and lower than 1300 meq O₂/kg, the most preferably greater than 1100 and lower than 1200 meq O₂/kg.

The formulation for topical use is particularly indicated for the use in the prevention and/or treatment of a cutaneous tumour, such as for example the malignant melanoma, the basal cell epidermoid carcinoma, and the spinocellular epidermoid carcinoma.

The formulation for topical use is moreover particularly indicated for the use in the prevention and/or treatment of a mucosal tumour, for example of the mouth, nose, anus, vulva or vagina.

According to an alternative preferred embodiment, the aforesaid formulation is for use by oral administration.

It has been found moreover that the formulation for oral use of the present invention is effective in preventing the formation of metastases, in particular in radioresistant subjects.

The present invention therefore further relates to the formulation for oral use of the present invention for use in the prevention of the formation of metastases, more preferably in a radioresistant human or animal mammal.

The formulation for oral use object of the present invention can consist of the aforesaid at least one ozonized oil or it can comprise the aforesaid at least one ozonized oil, preferably in a concentration, in weight percent with respect to the total weight of the formulation, from 1 to 5%, even more preferably from 2 to 3%.

According to a preferred aspect, the formulation of the present invention comprises a vehicle, compatible with the ozonized oil, preferably in an amount from 95% by weight to 99% by weight, more preferably from 97% by weight to 98% by weight, where the percentages refer to the total weight of the formulation.

According to a preferred embodiment, said vehicle comprises at least one sweetener and water.

Preferably, the sweetener is chosen from at least one saccharide, more preferably chosen from fructose, glucose, sucrose, and mixtures thereof; at least one fruit concentrate; at least one polyol; and at least one plant extract, more preferably stevia; and mixtures thereof.

In a preferred embodiment, the vehicle comprises a syrup and/ or honey.

Preferably, the syrup is a fructose syrup.

Preferably, honey is raw unprocessed honey.

The vehicle may further comprise one or more further ingredients chosen from lemon essential oil, acidifiers, citric acid monohydrate, preserving agents, sodium lactate, potassium sorbate, sodium benzoate, gum arabic, xanthan gum, natural or artificial flavourings (for example cherry flavour), emulsifying agents (polysorbate 80) and mixtures thereof.

The vehicle can also comprise osmotic water.

The vehicle can further comprise at least one pharmaceutically acceptable excipient.

Preferably, the formulation for oral use of the invention is in a form chosen from capsule and drink, more preferably a drink, even more preferably a syrup.

The term "syrup" here means an aqueous preparation containing at least one sweetener. The syrup is in an easily dosable fluid form, and does not require the use of coatings, in particular gastro-resistant coatings.

Preferably, the vehicle further contains a liposomal fraction. Said liposomal fraction can advantageously be used in order to increase the absorption level of the ozonized oil, since it favours the bioavailability and allows obtaining a more prolonged and gradual release of the active component.

Preferably, the formulation for oral use of the invention is in the form of a gastro-resistant capsule, for example with a silica-based coating.

In fact, silica carries out an adsorption action on the ozonized oil allowing a controlled release along the intestinal tract after passing the gastric barrier, rather than remaining in the form of a bolus.

In fact, ozonized oils tend to be largely neutralised in their action in the strongly acidic and therefore reducing gastric environment.

Advantageously, the formulation for oral use comprising ozonized oil of the invention has a high bioavailability, therefore it is able to reach the systemic circulation and therefore exert the therapeutic activity with respect to a malignant tumour. In fact, the particular formulation for oral use according to the invention, for example in the form of syrup, favours the bioavailability and absorption immediately after intake, starting from the very initial part of the digestive tract, including therein the oral and sublingual mucosa. For this reason the formulation for oral use of the present invention is characterized by the absence of gastro-resistant coatings which would prevent the rapid systemic absorption.

Furthermore, the formulation for oral use according to the invention, which is a rapid absorption formulation already in the initial tract of the digestive tract, makes the use of antioxidant (and therefore reducing) additives for preservation purposes unnecessary. In fact, these antioxidant additives interact with the active principle (ozone, oxidant), thus affecting the therapeutic properties of the formulation and the possibility of its absorption systemically. Therefore, the aforesaid peculiar characteristics make the formulation according to the invention particularly suitable for the induction of systemic and not only topical healing effects.

According to a preferred aspect of the present invention, the oral use for the treatment of a tumour is performed by oral administration of the formulation, preferably for a period of more than three days and less than sixty days, more preferably from ten to thirty days.

Preferably, the oral administration of the formulation is performed once or twice a day, more preferably twice a day.

According to a further preferred aspect of the present invention, the oral use for the prevention, in particular for the prevention of relapses, of a tumour is performed by oral administration of the formulation, preferably for a period of more than three days and less than sixty days, more preferably from ten to thirty days.

In the case of prevention of relapses, the administration of the formulation is preferably directed to the stem cells, which are particularly associated with the development of relapses. In fact, these cells are characterized by a very reducing intracellular environment that allows them to resist chemo/radiotherapy. The formulation of the present invention has the capability of modifying this environment to oxidative.

Preferably, the oral administration of the formulation is performed once or twice a day, even more preferably twice a day.

According to a further preferred aspect of the present invention, the oral use for the prevention of the formation of metastases is performed by oral administration of the formulation, preferably for a period of more than three days and less than sixty days, even more preferably from ten to thirty days.

Preferably, the oral administration of the formulation is performed once or twice a day, even more preferably twice a day.

Preferably, the oral administration is performed by administering an amount of ozonized oil from 0.1 to 0.5 ml per day per kilogram of body weight of the subject. This corresponds for example to a 7-35 ml dosage of ozonized oil per day in a patient of 70 kg of body weight.

More preferably, the oral administration is performed by administering an amount of ozonized oil from 0.2 to 0.4 ml, even more preferably from 0.25 to 0.35 ml per day per kilogram of body weight of the subject.

The formulation for oral use is particularly indicated for the use in the prevention and/or treatment of a liquid tumour, for example a hematopoietic tumour.

Furthermore, the formulation for oral use is particularly indicated for the use in the prevention and/or treatment of a tumour affecting the internal organs, for example colon, liver, pancreas, lung, breast, prostate, and the organs protected by the blood-brain barrier (including the other organs of the central nervous system, such as the brain, and the testicle). According to an alternative preferred embodiment, the aforesaid formulation is for use by administration by injection.

The formulation by injection of the present invention can consist of the aforesaid at least one ozonized oil or it can comprise the aforesaid at least one ozonized oil in a concentration, in weight percent with respect to the total weight of the formulation, of at least 90%, preferably of at least 95%, the most preferably 99%.

In a preferred embodiment, the formulation by injection object of the present invention comprises a pharmaceutically acceptable vehicle, compatible with the ozonized oil.

Preferably, the formulation by injection of the invention is in the form of an oil.

In a preferred embodiment, the formulation by injection of the invention further comprises at least one non-ionic surfactant.

Preferably, the surfactant is a polysorbate (polyoxyethylene glycol sorbitan alkyl esters) or SPAN (sorbitan alkyl esters).

Preferably, the surfactant is chosen from: polysorbate 80 (polyoxyethylene (20) sorbitan monooleate), polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), sorbitan monolaurate, sorbitan monopalmitate, sorbitan monostearate, sorbitan tristearate and sorbitan monooleate and mixtures thereof, more preferably polysorbate 80.

Thanks to this expedient, it is possible to make the ozonized oil water-dispersible, thus preventing the possibility that the injection of the ozonized oil by intramuscular or intravenous route leads to complications such as for example pulmonary or cerebral embolism.

In a preferred embodiment, the formulation of the invention by injection comprises aqua, polysorbate 80, ozonized peanut oil or sunflower oil, sodium chloride.

Preferably, the injection is by infiltration, phleboclysis, intravenous, intramuscular, or even by administration by porter (*port-à-cath*)*.*

According to a preferred aspect of the present invention, the use by injection in the treatment of a tumour is performed by injection of the formulation into the affected tissue, preferably for a period of more than three days and less than sixty days, more preferably from ten to thirty days.

Preferably, the injection of the formulation is performed once or twice a day, even more preferably twice a day.

Preferably, the administration by injection is performed by injecting an amount of ozonized oil from 0.5 to 5 ml, more preferably from 0.7 to 4 ml, even more preferably from 1 to 3 ml per day per cm³ of neoplastic mass.

The formulation by injection is particularly indicated for the use in the treatment of a solid tumour, such as for example a prostate, liver, lung, breast or colorectal tumour, particularly in the cases in which the tumour is in an advanced stage.

The formulation by injection according to the present invention can further comprise at least one pharmaceutically acceptable excipient.

According to a further preferred aspect, the formulation of the invention is for use by inhalation administration, for example in the form of an aerosol.

The formulation for inhalation use of the present invention can consist of the aforesaid at least one ozonized oil or it can comprise the aforesaid at least one ozonized oil, preferably in a concentration, in weight percent with respect to the total weight of the formulation, from 1 to 50%, even more preferably from 2 to 20%, the most preferably from 3 to 15%.

In a preferred embodiment, the formulation for inhalation use object of the present invention further comprises a pharmaceutically acceptable vehicle, compatible with the ozonized oil.

Preferably, the formulation for inhalation administration further comprises at least one non-ionic surfactant, as described above.

Thanks to this expedient, it is possible to make ozonized oil water-dispersible.

In a preferred embodiment, the formulation of the invention for inhalation use comprises aqua, polysorbate 80, ozonized peanut oil or sunflower oil, sodium chloride.

The formulation for inhalation use is particularly indicated for the use in the prevention and/or treatment of a tumour affecting the lungs (lung carcinoma) or the proximal part of the respiratory system (bronchi, larynx, pharynx).

Preferably, the prevention and/or treatment of the present invention comprise one or more among topical administration, oral administration, administration by inhalation route and administration by injection of the aforesaid formulation.

That is, it is possible to associate, for example, oral administration with topical administration and/or with the administration by injection.

It is also possible to associate topical administration with the administration by injection and/or oral administration.

Finally, it is possible to associate the administration by injection with oral administration and/or with topical administration.

With the appropriate association of the aforesaid methods of administration, it is possible to obtain, for example, both the tumour remission and the prevention of metastases.

The aforesaid formulations for oral use, for topical use and by injection can be used in the prevention and/or treatment of tumours, and/or in the prevention of the formation of metastases, and/or relapses, simultaneously or sequentially.

Preferably, the formulation is administered in association with a surgical intervention, more preferably a cryosurgery intervention aimed at removing the tumour, or in association with a cryosurgery intervention.

That is, the formulation can be administered by oral route, by inhalation route, applied topically, injected or combinations thereof, starting from a specific day before a surgical or cryotherapy intervention and up to a specific day thereafter. Alternatively, it can be administered by oral route, by inhalation route, applied topically, injected, or combinations thereof, starting from the day of a surgical intervention and up to a specific day thereafter, or starting from a specific day after a surgical or cryotherapy intervention.

Preferably, the formulation for oral use is administered in association with a radiotherapy session aimed at treating the tumour, more preferably in radioresistant human mammals or animals.

The formulation for oral use can be administered starting from a specific day before a radiotherapy session and up to a specific day thereafter, more preferably starting from a specific day before a radiotherapy session and up to the starting day thereof, even more preferably starting at least 20 days before a radiotherapy session.

It has been found in fact that the oral administration of the formulation starting before radiotherapy inhibits the formation of metastases in radioresistant subjects.

Preferably, the treatment of the tumour according to the present invention comprises the administration of the formulation of the invention until a U Cor/U Carr ratio is reached between anti-oxidizing species (U Cor) and oxidizing species (U Carr) in the patient's blood comprised between 4 and 12, more preferably comprised between 5 and 10, the most preferably comprised between 6 and 8.

Values lower than 4, in fact, if persistent over time, are indicative of an overdosage of the formulation and are therefore to be avoided because an oxidative damage can be caused in the patient's healthy cells, with consequent increased risk for the patient of cardiovascular diseases, such as heart attacks and strokes. Values above 12 are instead indicative of a treatment that is not very effective or not completed.

Preferably, the treatment of the tumours according to the present invention comprises the administration of the formulation of the invention until a U Cor/U Carr ratio is reached between anti-oxidizing species (U Cor) and oxidizing species (U Carr) in the patient's blood lower than 7, more preferably lower than 6, even more preferably lower than 5.

Preferably, the aforesaid ratio is equal to or greater than 4.

It has been observed in fact that in the case of a patient suffering from malign neoplasm, especially if aggressive and/or of large dimensions, the aforesaid U Cor/U Carr ratio is altered with respect to a healthy individual, in whom the normal value is 7 ± 1. In an oncological patient this value is well above the values of healthy individuals and values of 46 ± 7 are typically found.

The starting value of this parameter is variable among patients and depends on many factors, in addition to the presence of the tumour, including diet, age, lifestyle, other diseases, etc. Monitoring this parameter before and during treatment therefore allows setting up a personalised therapy for the patient, which allows the reduction of the neoplasm to be achieved more effectively while avoiding overdosage of the formulation.

The evaluation of the oxidative state, i.e. the determination of the aforesaid U Cor/U Carr ratio, can be performed by any method known in the field, for example by means of the FRAS test based on oxidation-reduction reactions of ferrous substrate with quantitative readings of the absorbance at 505 nm after reaction with the blood sample under examination. The FRAS test can be performed (a) for the evaluation of the anti-oxidizing species by the method described in Benedetti et al., Clin. Lab 59; 1091-1097, 2013; (b) for the evaluation of oxidizing species by the method described in Iannitti et al., J. Oral Path. Medicine, doi: 10.1111/j.1600-0714.2012.01143.x, 2012).

The test gives the quantification of: (a) total oxidizing species in equivalents of hydrogen peroxide molecules (1 U Carr = 0.08 mg H₂O₂ per 100ml); and (b) total anti-oxidizing species in equivalents of ascorbic acid molecules (1 U Cor = micromoles/litre of ascorbic acid). The test is non-invasive and is carried out by taking a micro-amount (50-100 ul) of peripheral venous blood by micro-puncture of the distal digital skin. It is possible to use tests that are similar to FRAS provided they are properly validated and compared to it.

Preferably, the treatment comprises one or more treatment sessions, spaced by a specific period of time, until stabilisation of the aforesaid desired value over time.

This specific period of time can be between 1 and 6 months, preferably between 1 and 3 months.

The value is considered to be stable over time when it remains substantially constant for at least six months, more preferably at least one year, even more preferably at least two years, the most preferably at least three years.

It is within the capability of the person skilled in the art to modulate the dosage and duration of the treatment, and its possible subdivision into one or more sessions, to achieve the pre-established target, based on the clinical evaluation of the patient with reference to the specific oncological pathology from which he suffers.

The present invention also refers to the prevention and/or treatment of a tumour, preferably a malignant tumour, which comprises the administration of the formulation of the invention in a human or animal mammal in need thereof.

The present invention also refers to the prevention of the formation of metastases and/or relapses which comprises the administration of the formulation for oral use of the invention in a human or animal mammal in need thereof, preferably a radioresistant human or animal mammal.

The topical use of ozonized oil is currently known for the treatment of cutaneous diseases and/or lesions. In particular, the use of ozonized oil is particularly suitable in the treatment of skin diseases and infections, also as regards chronic infections of cutaneous areas. It has now surprisingly been found that ozonized oil is effective in the prevention and treatment of the aforesaid tumours, particularly malignant tumours.

Without thereby wishing to be bound by any theory, it is hypothesized that the gaseous or aqueous preparations based on ozonizing species of the prior art are less effective than the formulation of the present invention in the prevention and/or treatment of tumours, in particular malignant ones, because the gaseous or aqueous form limits the action of the oxidizing effect to the the extracellular environment: the neoplastic cells are in fact surrounded by a lipophilic membrane that prevents the direct entry of gas or water.

On the contrary, with the formulation of the present invention, comprising an ozonized oil, effective and long-lasting results are obtained.

In fact, the oil, by penetrating into the mitochondrial membranes of the neoplastic cell is able to activate the apoptosis by oxidation and thus cause the death of the neoplastic cell.

The *in vitro* tests show in fact that the formulation of the invention is able to exert a powerful anti-cancer effect at the cellular level and have elucidated the mechanism of action of the formulation of the present invention, which is based on the penetration of the ozonized oil into the cell cytoplasm due to its capability of oxidizing the cell membranes, including the plasmalemma, which delimits the cell from the external environment. Once penetrated into the cytoplasm, the ozonized oil is trapped in vacuoles for a short time. However, the membranes of these vacuoles are rapidly oxidized by the ozonized oil, and then the oil diffuses into the cytoplasm, thus exerting its action.

The formulation of the present invention is therefore characterized by a unique capability of penetrating inside the neoplastic cells thanks to the damage it also induces on the external cell membrane. Given the purity (that is, the high concentration of ozonides) and the high stability of the ozonized oil, and consequently of the formulation of the invention, the penetration does not compromise the pro-oxidant capabilities which are then directly expressed in the intra-cellular site. This aspect is peculiar in that other formulations of ozonizing species are not able to have this effect as they are neutralised by the anti-oxidants in the extra-cellular or intra-cellular environment, both particularly rich in anti-oxidant defences, especially in neoplastic stem cells.

Cancer is a systemic disease of the body. The neoplastic mass cannot independently develop adequate trophic support structures for its own development. Therefore a characteristic of carcinoma is the presence of a conspicuous infiltrate of inflammatory macrophage cells that, completely incapable of contrasting the neoplastic growth, support it by supplying oxygen, metabolites and neo-vessels. These cells are defined as Macrophages Associated with Tumours. Today the degree of inflammation represents the most predictive prognostic index of the inauspicious development of the neoplasm. Cancer is therefore more effectively contrasted with the inclusion of an oral formulation capable of contrasting the inflammation systemically.

It has been observed that the formulation of the invention is able to exert anti-inflammatory effects without inducing immuno-suppression, as demonstrated by the *in vitro* and *in vivo* tests.

Without wishing to be bound by any theory, it is hypothesized that the mechanism underlying this phenomenon is the inhibition of the macrophage oxidative burst. Macrophages, responsible for the non-specific cell-mediated tissue immunity, operate by releasing reactive oxygen species and inflammatory cytokines in the tissue environment that are able to neutralise bacteria if present; in the absence thereof, however, inflammatory phenomena develop following the macrophage activation that assume, as in the case of cancer, a pathogenic relevance. The formulation of the invention inhibits the macrophage oxidative burst through negative feedback; in fact the presence of an extra-cellular environment already rich in ozonic oxidizing species blocks the release of further oxidizing species by the macrophages, thus inhibiting the activation and consequent inflammation.

It is also believed that the selective effect of the ozonized oil of the present invention, with a high content of ozonides, of killing neoplastic cells and not the normal differentiated cells is due to the fact that the mitochondrion in the neoplastic cell is inactive, both from the metabolic point of view and from the point of view of the activation of the programmed cell death (apoptosis) by intrinsic route by release of cytochromes and calcium in the cytoplasm. This characteristic differentiates the neoplastic cell from a healthy cell.

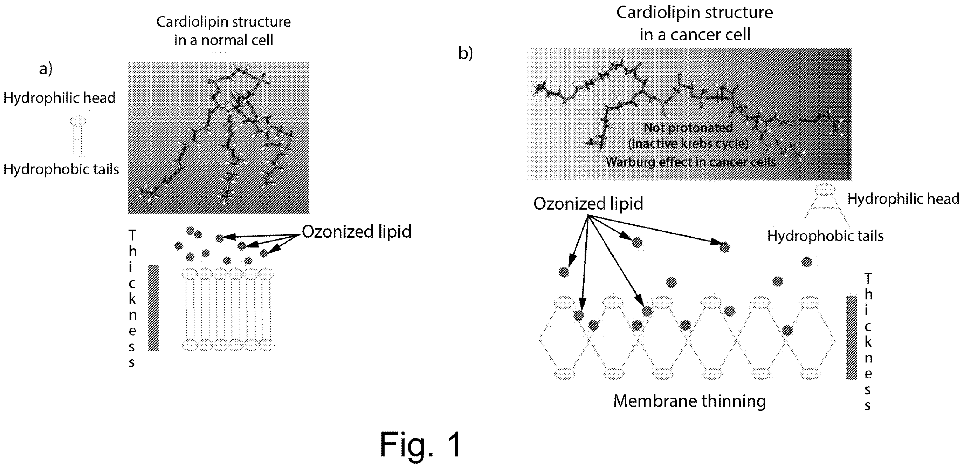

The mitochondrial membrane is mainly composed of phospholipids, the most important of which is cardiolipin, which is arranged so as to form the typical lipid bilayer of the external mitochondrial membrane by combining the hydrophobic tails of two molecules and causing the hydrophilic heads to project into the cytoplasmic hydrophilic environment outside the mitochondrion and into the hydrophilic environment of the matrix inside the mitochondrion. The structure of cardiolipin is however extremely dependent of its bond with cytochrome c, a fundamental active component of oxidative phosphorylation. Cardiolipin, in particular, is arranged as described above in the presence of a bond with the functioning cytochrome c, that is, in a healthy tissue. The bilayer formed by the cardiolipin is in this case thick, tight and symmetrical and with a continuity of the hydrophilic heads. This arrangement prevents access to the hydrophobic tails of the peroxidant free radicals carried by the ozonized oil of the present invention. The healthy cell is therefore resistant to the cytocidal effects of the ozonized oil of the present invention (see Figure 1a).

Conversely, in the neoplastic cell, cardiolipin is found in the absence of functioning cytochrome c. This results in a divarication of the hydrophobic tails, giving rise to a thinned membrane and gaps between the hydrophilic heads. In this situation, the peroxidant free radicals carried by the ozonized oil of the invention have access to the hydrophobic tails and the cell is sensitive to the cytocidal effects of the ozonized oil of the present invention (see Figure 1b).

The efficacy of the formulation of the present invention may also depend on other factors such as its purity, the absence of antioxidants, and its high bioavailability.

In particular, in the case of the embodiment of the invention which involves the association of the formulation for oral use and the formulation for use by injection, it has been found that this association allows obtaining direct cytopathic effects by infiltration in the neoplastic mass, and therefore improved efficacy.

The formulation for oral use of the invention, as mentioned above, is in fact rapidly absorbed by systemic route. This aspect represents an absolute peculiarity with respect to low-ozonide ozonized oils present on the market which are not able to be absorbed systemically or that are sometimes even complexed with anti-oxidants for the purpose of stabilisation and preservation that affect their pharmacological effects.

In a preferred embodiment, the content of ozonides in the oil is greater than the levels ever reached in the prior art (10-30 meq O₂/kg of oil). This oil is particularly stable and powerfully oxidizing, especially in the intracellular environment of the neoplastic cell

The formulation of the present invention therefore allows stabilising very high quantities of ozonic oxidizing species (ozonides) in a lipophilic complex allowing it to penetrate inside the neoplastic cell and activate in this site the mechanisms of autonomous cell death (apoptosis) without damaging the surrounding healthy tissues.

The formulation of the present invention is characterized by a duration of the therapeutic effect of at least six months, preferably at least one year, more preferably at least two years, even more preferably at least three years, the most preferably at least five years.

The formulation for oral use of the present invention finds particular use in the prevention of the formation of metastases and/or relapses.

In particular, when oral administration of the formulation is associated with radiotherapy in radioresistant subjects, it is observed that the formulation is effective in preventing the formation of metastases. In fact the low oxygen availability (hypoxia) is the main mechanism that triggers the migration of neoplastic cells from the primary site (metastasis). The invention presented here, by increasing the availability of oxygen at the site of the primary tumour, inhibits the activation of the metastatization process.

The high content of ozonides is also fundamental for the personalisation of the therapeutic dose. In fact, drug doses must be individualised based on the type of subject treated and the existing neoplastic pathology. The wide range of ozonides that the formulation makes it possible to have allows individualising the therapeutic dose with a precision that has never been reached before. This personalisation is carried out based on the quantitative evaluation of the ratio between oxidizing and anti-oxidizing species in the plasma of the treated subject, as illustrated above. Finally, the formulation of the present invention is characterized by a high tolerability and scarcity of undesired side effects, a very peculiar situation for a drug capable of killing cancer cells. This characteristic is due to the big difference that exists between normal and neoplastic cells in their capability of contrasting oxidative damage, especially when it occurs in the intracellular environment.

Further features and advantages of the present invention will be apparent from the following detailed description.

### Brief description of the figures

Figure 1 shows the structure of cardiolipin in normal cells (Figure 1a) and in a neoplastic cell (Figure 1b).

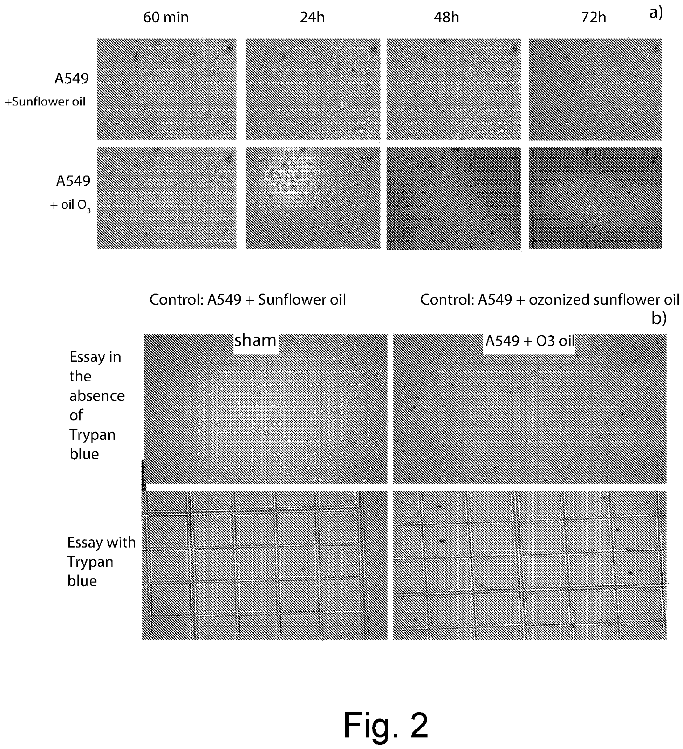

Figure 2 shows the results of an *in vitro* cell assay for determining the anticancer effect of an ozonized sunflower seed oil of the invention on a A 549 cancer cell line. Figure 2a is an optical microscope image of the cell line after 60 min, 24 hours, 48 hours and 72 hours of exposure to the ozonized sunflower seed oil of the invention. Non-ozonized sunflower seed oil was used as a comparative control. Figure 2b is an optical microscope image showing selective cell staining with Trypan blue, a selective stain of dead cells only.

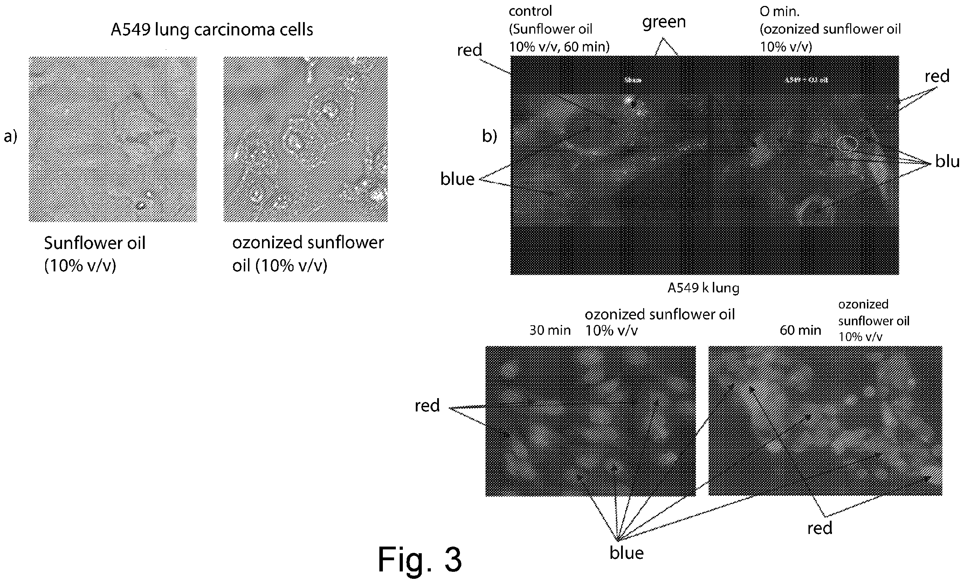

Figure 3 shows the results of an *in vitro* cell assay using normal sub-cellular and trichrome fluorescence microscopy to determine the mechanism of action at the intracellular level in the neoplastic cell. Figure 3a shows an optical microscope scan of cells of the A549 cell line following exposure to the ozonized sunflower seed oil of the invention, at a concentration of 10% v/v in Dulbecco Modified Eagle Medium (DMEM), for two hours: the intracellular penetration of the ozonized oil into the cytoplasmic site is detected (clear reflective vacuoles). Non-ozonized sunflower seed oil was used as a comparative control. Figure 3b shows the trichrome fluorescence microscopy images of cells of the A549 cell line following exposure to the ozonized sunflower seed oil of the invention for 0, 30 minutes and 60 minutes with staining. Non-ozonized sunflower seed oil was used as a comparative control. The stainings indicate: (a) red: ozonized oil; (b) blue: nucleus; (c) green: cytoplasmic structures and specifically mitochondria. The penetration of the oil into the cytoplasmic site is confirmed.

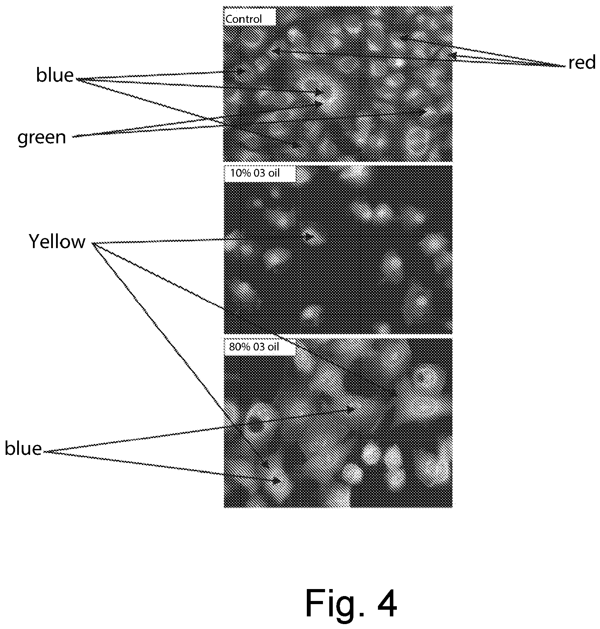

Figure 4 shows further fluorescence microscope images of cells of the A549 cell line exposed to a control sunflower oil and an oil containing 10% and 800 of ozonized sunflower oil with trichrome staining with superimposed channels (merging). The yellow stain indicates the superimposition between red and green. This superimposition occurs only in cells treated with ozonized oil and not in the control; it indicates that the oil (red) reaches and damages the mitochondria (green) (Example 3).

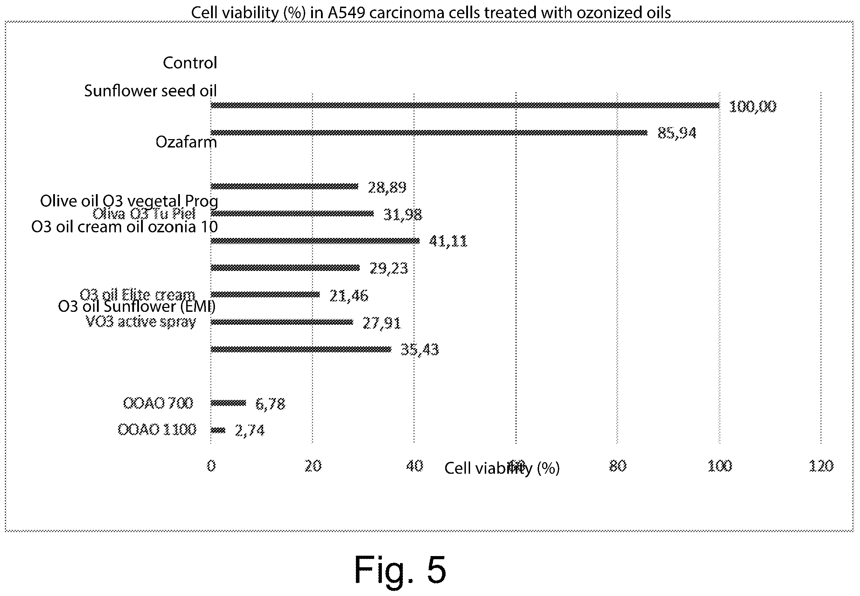

Figure 5 shows a comparison between cell viability values in A549 cells following treatment with different oils according to Example 4.

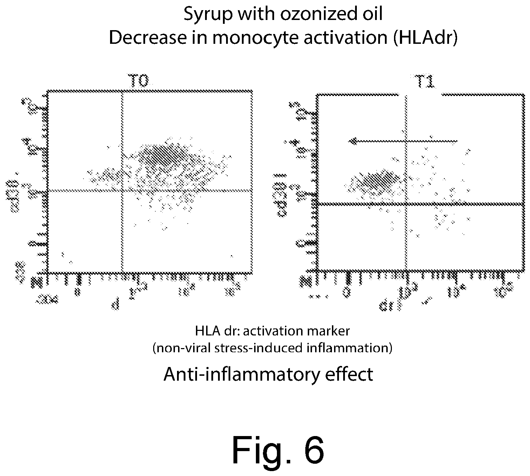

Figure 6 shows the results of an *in vivo* cell assay to determine the anti-inflammatory power of the formulation of the invention. The graph shows the decrease of the HLAdr macrophage activation marker in two subjects treated with the syrup of the invention, measured by citofluorimetry.

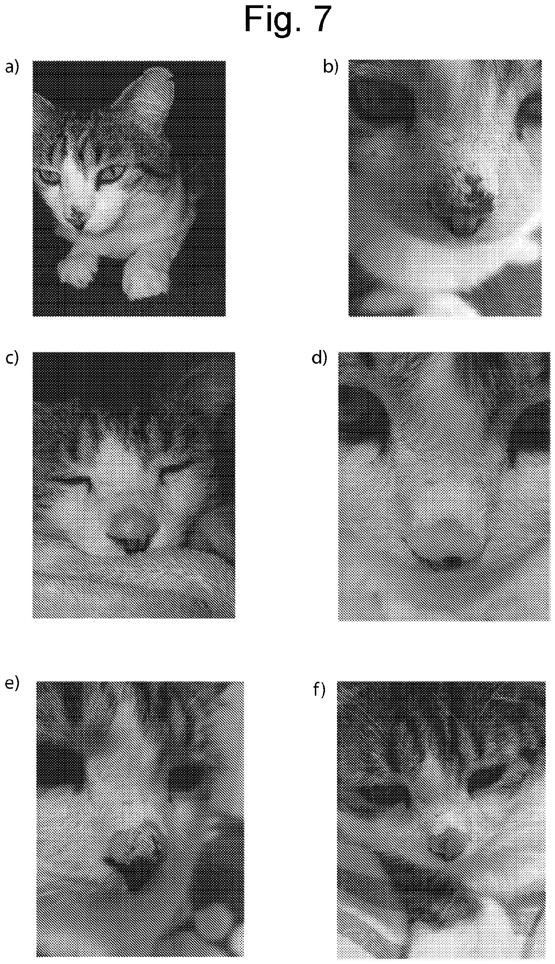

Figure 7 is a series of photographs showing the healing progress of a cat suffering from squamous cell epidermoid nasal carcinoma following treatment with the formulation of the invention. In particular, the figures show the state of the cat before treatment (Figure 7a), 24 hours after the intervention (Figure 7b), 72 hours after the intervention (Figure 7c), 7 days after the intervention (Figure 7d), 10 days after the intervention (Figure 7e), and three weeks after the intervention (Figure 7f) (restitutio ad integrum).

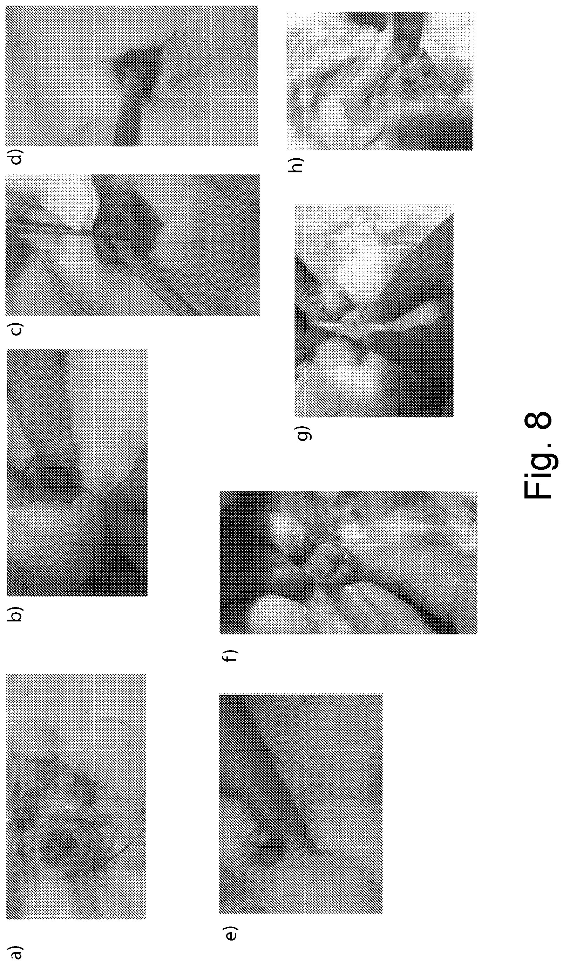

Figure 8 is a series of photographs showing the healing progress of a rabbit suffering from ulcerative squamous rectal carcinoma following treatment with the formulation of the invention and cryotherapy intervention, before the intervention (Figure 8a-d), 7 days after the intervention (Figure 8e), 10 days after the intervention (Figure 8f), four weeks after the intervention (Figure 8g) (restitutio ad integrum), and seven months after the intervention (Figure 8h) (persistence of restitutio ad integrum).

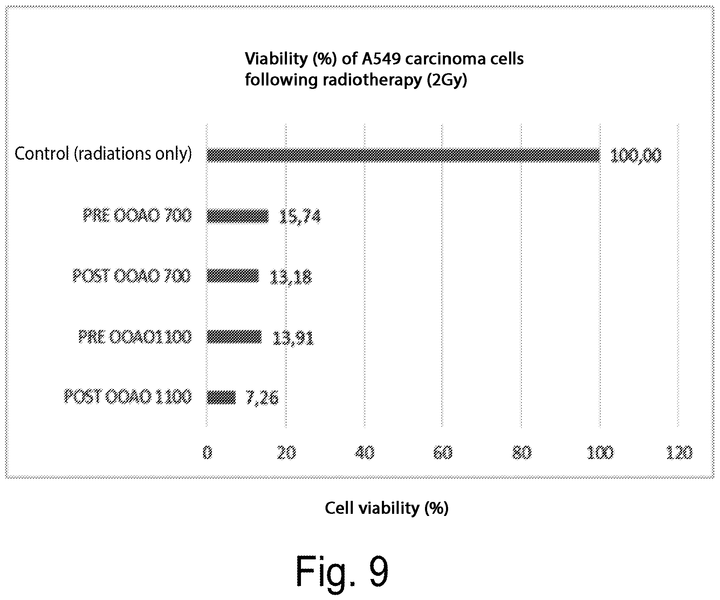

Figure 9 shows the results of the viability test of A549 tumour cells following exposure to the ozonized oil of the invention at 700 ("OOAO 700") or 1100 ("OOAO 1100") ozonides, administered before ("PRE") or after ("POST") radiotherapy (2Gy). OOAO increases the capability of radiations to kill neoplastic cells from 7 to 14 times. Post-treatment with OOAO1100 is the most effective therapeutic situation.

### Detailed description of the invention

The present invention will now be further illustrated by means of some embodiment examples as shown below.

### EXAMPLE 1

### Preparation of the formulation in the form of a syrup

The following formulation was prepared in the form of a syrup:
2.5% ozonized sunflower seed oil with a content of ozonides equal to 700 meq O₂/kg and the remaining 97.5% comprising an aqueous solution of fructose, acidifier, monohydrate citric acid, preserving agents, sodium lactate, osmotic water, potassium sorbate, cherry flavour, emulsifying agent (polysorbate 80); wherein said percentages refer to the total weight of the formulation.

### EXAMPLE 2

### In vitro assay of carcinoma cells

An ozonised sunflower seed oil with a content of ozonides equal to 700 meq O₂/kg was assayed on highly undifferentiated and aggressive human lung carcinoma cells (A549 cell line). Non-ozonized sunflower seed oil was used as a comparative control. The treatment consisted of the contact of the cells with the oil for two hours, followed by washing. The experiment was repeated in quadruplicate. Cells not treated or treated with non-ozonized sunflower seed oil rapidly grew reaching the confluence in the growth plate after 72 h. The cells treated with the oil of the invention showed a difficulty in growth in the first 24 hours, after which they rapidly underwent cell death culminating at 72 hours with the disappearance of the cell growth bed and the mere presence of dead cells and diffuse apoptotic bodies in the supernatant. Figure 2a shows the results obtained in an experiment.

The death of cells treated with ozonized oil was also demonstrated by selective staining with Trypan blue according to a standard protocol in the sector (Figure 2b).

In particular, the cells of the A549 line were seeded in a 75 cm² flask for cell cultures and treated with ozonized sunflower oil (General Service, Spain) at a concentration of 10% v/v in DMEM. The control was carried out by treating the A549s in a 75 cm² flask with sunflower oil (EMI) at the same concentration. Four hours after treatment, an aliquot of cells was taken from each flask, placed on a microscope slide and stained with trypan blue, as reported by Strober et al. (Strober W., Trypan Blue Exclusion Test of Cell Viability. Curr Protoc Immunol. 2015 Nov 2;111:A3.B.1-3).

### EXAMPLE 3

### Mechanism of action at the intracellular level in the neoplastic cell

The dynamics with which the product of the invention induces cancer cell death were examined by means of normal subcellular and trichrome fluorescence microscopy in which the nucleus was stained in blue, the mitochondria and the cellular membranes in green, the sunflower seed oil and the ozonized oil in red.

8×10⁴ cells of the A549 line were seeded on a microscopy coverslip (20 × 20 mm). On the following day, the cells were treated with ozonized sunflower oil (content of ozonides equal to 700 meq O₂/kg) (General Service, Spain) at different concentrations (10% and 800 v/v in DMEM) for two hours. The control was carried out using not ozonized sunflower oil (EMI Supermercati, Italy) at the same concentration as the treatment and for the same duration of time. Figure 3a shows the intracellular penetration into the cytoplasmic site of the 10% v/v ozonized oil (clear reflective vacuoles).

In order to remove the oils, after 24 hours the cells were washed with a solution containing 0.1% of Nonidet P-40 (Sigma-Aldrich, Milan, Italy) in PBS (Phosphate Buffer Saline, Euroclone, Milan, Italy) and subsequently with PBS alone. After the washings, the cells were fixed with 4% Paraformaldehyde for 10 minutes at 37°C and finally stained with DIoC6, Nile Red, Rhodamine and DAPI, as reported by Chen et al., Tarnowski et al., Koning et al., Johnson et al. (Chen W et al., A high throughput Nile red method for quantitative measurement of neutral lipids in microalgae. J Microbiol Methods. 2009 Apr; 77(1):41-7; Tarnowski BI et al., DAPI as a useful stain for nuclear quantitation. Biotech Histochem. 1991;66(6):297-302.; Koning AJ et al., DiOC6 staining reveals organelle structure and dynamics in living yeast cells. Cell Motil Cytoskeleton. 1993;25(2) :111-28; Johnson LV et al., Localization of mitochondria in living cells with rhodamine 123. Proc Natl Acad Sci USA. 1980 Feb;77(2):990-4).

It was observed that the non ozonized sunflower seed oil has only minimally penetrated into the cells (cytoplasm) where it has been compartmentalized into small well-defined vacuoles. Conversely, the ozonized oil has penetrated abundantly into the cytoplasm, probably due to its peculiar capability of oxidizing the cellular membranes including the plasmalemma that delimits the cell from the external environment. Once it penetrated into the cytoplasm, the ozonized oil was initially trapped in vacuoles; however, the membranes of these vacuoles were rapidly oxidized and the oil diffused in the cytoplasm by generating a red halo that was superimposed on the green of the membranes and the intracellular organelles (mitochondria). The results relative to 10% v/v ozonized oil are reported in Figure 3b.

These events culminated at 24 hours and were followed by the death of the cells treated with the oil of the invention. It is therefore evident that the ozonized oil of the invention penetrates inside the neoplastic cells where it generates a cytoplasmic oxidative damage. The organelle most sensitive to this event is the mitochondrion. The oxidation of the mitochondrial membranes of the neoplastic cell is able to activate apoptosis and therefore the death of the neoplastic cell.

The state of the mitochondrial function in cells treated with ozonized oil was then analysed by evaluating the release of calcium by mitochondria using red fluorochrome (rhodamine 2) as a direct indicator of mitochondrial damage.

The end point was measured in A549 neoplastic cells treated with sunflower oil (control) or with ozonized oil at the 10% v/v and 80% v/v dose with respect to the culture medium. An increase in the release of intracytoplasmic calcium in a single dose dependent of the amount of ozonized oil used was detected.

Then, the selective staining of the mitochondrial membranes was performed by means of green stain (DiOC6) verifying the variation of signal intensity in the control with respect to the cells treated with ozonized oil. A marked decrease in signal intensity in the cytoplasm (where the mitochondria are located) was observed in the cells treated with ozonized oil; this result is indicative of a damage of the mitochondrial membrane induced by the treatment with ozonized oil. Large cytoplasmic lipid vacuoles were also observed in the treated cells. These vacuoles contain oxidized lipids that are not catabolized by the cell, which therefore takes on the appearance of a 'foaming cell' which characterizes cells that cannot catabolize lipids due to the high level of mitochondrial damage. The presence of such vacuoles in the cells treated with ozonized oil is therefore a further evidence that the action of the formulation of the invention is exerted preferentially and directly on the mitochondrial membrane.

The digital superimposition of the images analysing the release of intracellular calcium and the mitochondrial membrane damage was then performed (Figure 4). The images take on a yellow stain that derives from the almost perfect superimposition of red with green. This result indicates that intracellular calcium (red) is released precisely by the mitochondrial (green) membranes damaged by the ozonized oil. In fact, this superimposition (yellow stain) does not exist in the control cells where the mitochondrial membranes are not damaged and therefore are stained in green and where no calcium is released by the mitochondria.

This result demonstrates how the mechanism of therapeutic action of the formulation of the invention is the intracellular induction of a mitochondrial damage with consequent activation of the intrinsic cellular apoptosis.

### EXAMPLE 4

### Comparison tests of different oils on cell viability

A comparative experiment was performed to evaluate the peculiarity of the effect of the oil of the formulation of the present invention with respect to other ozonized oils.

A549 anaplastic carcinoma cells were grown in the presence of various low-ozonide ozonized oils or of oils of the present invention at 700 ("OOAO 700") and 1100 meq O₂/kg ("OOAO 1100"); their capability of inducing cell death was evaluated by vital staining with crystal violet, according to the following protocol.

Cells of the A549 line were seeded in 96-micro-well flat-bottomed plates at a density of 6×10³ cells per well in 100 µl of DMEM culture medium (Sigma-Aldrich, Milan, Italy) . On the following day, 10 µl of ozonized sunflower oil (General Service, Spain) were added to each well and the cells were incubated at 37°C in a CO₂ incubator for 24 hours. The control was carried out using sunflower oil (EMI Supermercati, Italy) at the same concentration as the treatment and for the same duration of time. After incubation, the cells were initially washed with a solution containing 0.1% of Nonidet P-40 (Sigma-Aldrich, Milan, Italy) in PBS (Euroclone, Milan, Italy) and subsequently with PBS. Cell viability was evaluated by staining with crystal violet as reported by Feoktistova et al., Crystal Violet Assay for Determining Viability of Cultured Cells. Cold Spring Harb Protoc.; 2016(4) :pdb.prot087379]. The results were read in a microplate photometer (Multiskan FC, Thermo Scientific) at a wavelength of 570 nm.
The following preparations were evaluated with respect to the control not treated or treated with non-ozonized sunflower oil: VO3 active spray (Benedetti & Co Biosolutions Srl, Brescia, Italy), Ozonized olive oil (Vegetal Progress Srl, Turin, Italy), EMI sunflower oil (EMI Supermercati, Italy), Cream oil Ozone Elite (ECS Elitegroup), Cream oil Ozonia 10 (Innovares Srl, Italy), Olive oil O3 Tu Piel (Qualified naturopath, UK), Oil Ozofarm (Ozofarm, Parma, Italy), oil of the invention at OOAO 700 and OOAO 1100.

The results obtained are reported in Figure 5 indicating for each preparation the amount of still viable cells after treatment (percentage with respect to the untreated control, 100% viability). All experiments were replicated 8 times.

The results obtained indicate that cell survival decreases in the presence of ozonized oils with respect to the controls. However, none of the ozonized oil preparations of the prior art is able to lower the percentage of surviving cells below 20%. The only preparations capable of achieving this result are OOAO 700 and OOAO 1100. In fact the percentage of surviving cells is only 6.78% in OAOO 700 and the minimum observed value drops by 2.7% in the case of OOAO 1100.

### EXAMPLE 5

### Determination of the anti-inflammatory power of oil-based ozonized syrup

The anti-inflammatory power of the oil-based ozonized syrup of Example 1 was evaluated *in vivo* by evaluating in a comparative fashion at the times T0 (before the administration of the formulation of the invention) and T1 (at the end of the study) lymphocyte sub-populations in two treated volunteers, by flow citofluorimetry. The subjects (both healthy and 70 kg of body weight, 54 years of age) were treated with 2.5% ozonized syrup for 1 week at a dose of 12 ml twice a day between meals. Pro-inflammatory activation of macrophages and lymphocytes was evaluated in peripheral blood before and after treatment by citofluorimetry. The results obtained are reported in Figure 6 which shows a scatter plot of leukocyte distribution according to the marking with CD38 (leukocyte marker, vertical axis) and with HLAdr (marker of macrophage activation, horizontal axis). The Figure indicates the marked decrease in T1 with respect to T0 of macrophage activation markers (HLAdr) (arrow); no change was observed instead in lymphocyte sub-populations responsible for protective immunity.

These results indicate that the formulation in question is able to exert anti-inflammatory effects without inducing immune suppression.

### EXAMPLE 6

### Treatment of differentiated human keratinocytes

In order to evaluate the safety of the formulation in question and its inability to induce cytopathic effects in healthy cells, human differentiated keratinocytes were treated with ozonized sunflower seed oil (700 meq O₂/kg) according to the experimental protocols already described in the Examples 2 and 3. The results were examined at 48 and 72 hours without detecting any alteration of cell viability or any presence of cytopathic effects.

The safety of the formulation was also evaluated *in vivo* in the two volunteers (of Example 5) treated with the syrup, analysing the traditional blood chemistry parameters: the absence of any alteration of the basal physiological state was observed.

### EXAMPLE 7

### In vivo clinical case

An *in vivo* experimentation was then carried out in the animal to verify whether the therapeutic effects observed in the experimental models described so far were transferable to *in vivo* situations. Compassionate treatments were carried out under veterinary control in animals suffering from serious spontaneous oncological diseases for which there are no therapies with recognized efficacy. Some examples of the results obtained are reported below.

CASE 1. 8-year-old male cat with histologically confirmed squamous cell epidermoid nasal carcinoma (Figure 7a). This carcinoma is known for its aggressiveness and its rapid development in this animal so as to usually require the prompt euthanasia of the same. Being informed about the rapid evolution of the disease, about the local and systemic side effects and the costs of radiotherapy, about the "demolition" effects of a "conventional" surgery, the owners opted for the administration by infiltration, by oral route and by local application of ozonized oil.
The protocol followed in this clinical case was:
Infiltration of OOAO 700 (700 meq O₂/kg), 1 ml per session (4 sessions, 1 session every 5 days for a total of 4 sessions) in multiple topical injections (6 per session) in the neoplastic mass, on the affected part, for 20 days. Oral administration, starting from the same day of the first infiltration, of ozonized sunflower oil (700 meq O₂/kg, "OOAO 700"), at the dosage of 5 drops in the morning and in the evening in the wet food on which the subject was usually fed (can or bag of wet food for adult cats) for 21 days. Locally on the part affected by the lesions, a generous amount of OOAO 700 sunflower oil was applied twice a day, starting from the same day of the first infiltration, sufficient to cover it completely, including the perilesional skin, an application that was repeated for 21 days.

24 hours after the start of treatment, the onset of the necrosis of the area where the neoplasm was present was noted (Figure 7b).

After 72 hours, the eschars had softened and could easily be removed by the owner (Figure 7c).

After seven days the eschars were completely eliminated. A large ulcer persisted at the point of contact of the probe with the skin of the nose (Figure 7d).

After 10 days the almost complete healing of the ulcer was noted with neo-epithelialization of the whole tip of the nose (Figure 7e).

At the end of the third week a complete "restitutio ad integrum" of the surface of the tip of the nose took place. The owners, given the apparent state of well-being of the subject, decided to stop all therapy (Figure 7f).

The therapy was thus interrupted, the animal placed under observation for 8 months without finding any relapse of the neoplastic disease which was therefore eradicated. To date, after more than ten months, the subject is in excellent health and shows no signs of tumour regrowth. The owners report a complete return to normal life without any olfactory deficit.

### EXAMPLE 8

### Second In vivo clinical case

CASE 2. 5-year-old rabbit with histologically confirmed squamous ulcerative rectal carcinoma (Figure 8a). The lesion was highly malignant, inflamed and bleeding.

Given the delicateness of the area and the surgical risk of faecal incontinence, it was decided to perform cryosurgery with protective use of the healthy area with ozonized oil.

Before the intervention 7 days of oral intake of 5 drops of ozonized sunflower oil (OOAO 700, 700 meq O₂/kg) in the morning and in the evening were started which were continued up to 10 days after cryotherapy. On the day of the intervention the preparation of the subject was carried out by greasing the whole affected area very carefully with oil (Figure 8b), so as to completely cover the affected area, including the perilesional skin. An infiltration was also performed after the intervention (OOAO 700, 1 ml per session in multiple topical injections in the neoplastic mass, as already described in Example 1) on the affected area. Figure 8c shows the execution of the surgery while Figure 8d shows the complete freezing of the neoformation. The topical application and the oral administration were continued up to 10 days after the intervention. On the seventh day after the intervention the presence of necrotic material was still noted and the area still appeared to be hyperaemic (figure 8e). Ten days after the intervention, there was no necrotic material and the oedema of the part and the surrounding neovascularised inflammatory halo were visibly reduced (Figure 8f). On the twenty-eighth day after the intervention, the complete restitutio ad integrum of the area affected by the neoplasm was observed (Figure 8g).

To date (11 months later) no signs of local regrowth or metastasis are present and the subject does not show any problems during defecation or urination (Figure 8h).

### EXAMPLE 9

### Case 3

### Human clinical case

A 93-year-old female patient with histopathologically confirmed malignant spino-cell epidermoid carcinoma. The neoplasm was located in the scalp in the parietal region and showed a character of extreme invasiveness and rapid progression *ab initio.* In fact, the brain case was invaded in just a few weeks with consequent parietal osteolysis. The neoplasm quickly continued its progression penetrating inside the skull and approaching to the arachnoid. These data were demonstrated by computerized axial tomography. The neoplasm appeared on the outside of considerable size so as to cover the entire cranial vault with noticeable not only endophytic (inside the skull) but also exophytic (protrusion outside the skull of the neoplastic mass) growth. The neoplasm appeared as highly malignant characterized by a high level of anaplasia, rapid progression, presence of neoplastic ulcers of significant size, a noticeable inflammation of the perilesional margins and total absence of phenomena of repair from granulomatous tissue in the areas surrounding the neoplastic ulcer. The patient was suffering and feverish.

Not being able to intervene surgically due to the extension of the neoplastic mass and the old age of the subject, it was decided to intervene with regional palliative radiotherapy with 8 sessions of 2 Gy each. From direct experience, this approach was expected to be purely palliative by the radiotherapists involved, as this type of cancer is very little responsive to radiotherapy. It was therefore decided to use ozonized sunflower oil at 1100 ozonides ("OAOO 1100") by local application on the carcinomatous ulcer.

Therefore, the problem of the moment of application of OAOO 1100 with respect to radiotherapy arose, namely if the product had to be applied before or after treatment with gamma radiations. For this purpose, an *in vitro* experiment was performed in which anaplastic A549 carcinoma cells were exposed to ionizing radiations (2 Gy) both under pre-treatment and post-treatment condition with OOAO 1100 and OOAO 700 (ozonized sunflower oil at 700 ozonides). Cell survival was therefore evaluated comparatively by means of the crystal violet test with respect to control cells exposed to radiations but not treated with ozonized sunflower oil. The experiment was replicated 16 times in multi-plate for a total of 48 experiments (3 experimental conditions x 16 replicates). The results obtained are reported in Figure 9.

The results obtained indicated a strong radiosensitizing effect of OOAO 700 and even more of OOAO 1100. The maximum effect found was that of OOAO 1100 when administered after exposure to gamma rays. From a mechanistic point of view, this effect is in line with the activation of intrinsic mitochondrial apoptosis activated by the ozonized oil in cells that have undergone genotoxic radiation damage. It was therefore decided to use OOAO 1100 in a post-treatment regime, i.e. at the end of each radiotherapy session. Therefore, at the end of the first radiotherapy session, OOAO 1100, gelled at 4°C, was placed on the neoplastic lesion by means of a glass depositor and the ulcer coated with gauze and hydrophobic bandage.

The treatment was continued for a total of 8 consecutive sessions with 2 days interval between each of them. The medication with OOAO 1100 was renewed at the end of each session. Therefore OOAO 1100 was left in place to act for 48 hours during the interval periods between sessions.

Macroscopic analysis showed clearly a strong decrease in the size of the neoplastic mass both in terms of its amplitude and its depth. The formation of repair granulation tissue at the margins of the lesion was also noted.

Radiotherapy was then stopped and only topical treatment with OOAO 1100 was continued. Subsequently the neoplasm did not resume growth but continued its regression.

These data substantiate the specific inhibitory effect of OOAO 1100 against the tumour also in the absence of radiation treatment. At the end of the follow-up, only a soft eschar of exudative material resulting from the colliquative necrosis of the neoplastic tissue persisted.

To verify whether the topical application of OOAO 1100 had had a systemic effect on the oxidative balance, free radical analysis was performed on blood plasma before (T0) the beginning of treatment with OOAO 1100 and at its end (T1) using Fras (Free Radical Analysis, Fras 4 Evolvo, H&D, Parma Italy)). In T0 the parameters were dramatically altered with a particularly low oxidizing species value, that is of 38 U carr (normal range 250-280). In parallel the antioxidants were strongly increased with a value of 8318 U cor (normal range 2200-2800). The antioxidant/oxidant balance (U cor/U carr ratio) was therefore of 219 (normal range 7-10). These results demonstrate that the presence of neoplastic masses of significant size imply the strong decrease in the level of systemic oxidative damage. In T1 the values change as follows: 201 U carr, 7544 U cor, 37 U cor/U carr. Therefore an increase of 530% of the oxidizing species was observed, a 13% decrease of the antioxidants, a 583% decrease of the U cor/U carr ratio. These values indicate that the regression of the neoplastic mass is related to the variation of the oxidative balance induced by the treatment with OOAO.

At the end of the treatment the patient's state of health was good and no longer suffering or feverish. Undesirable effects, both subjective and objective, that can be related to treatment with OOAO 1100 or exposure to radiation therapy were never found during treatment.

## Claims

1. A formulation comprising at least one ozonized oil for use in the prevention and/or treatment of a tumour, preferably a malignant tumour, wherein said at least one ozonized oil has a content of ozonides from 500 to 1500 meq O₂/kg.

2. The formulation for use according to claim 1, wherein said formulation is for use by administration chosen from oral, topical, by injection, by inhalation and combinations thereof.

3. The formulation for use according to claim 1 or 2, wherein said at least one ozonized oil has a content of ozonides from 600 to 1400 meq O₂/kg, preferably from 700 to 1300 meq O₂/kg.

4. The formulation for use according to any one of the preceding claims, wherein said ozonized oil is a vegetable oil that has undergone an ozonizing process, preferably chosen from: sunflower oil, olive oil, peanut oil, argan oil, grapeseed oil, jojoba oil, soybean oil, corn oil, palm oil, cottonseed oil, rapeseed oil, coconut oil, castor oil, linseed oil, borage oil, evening primrose oil, and mixtures thereof, preferably sunflower oil.

5. The formulation for use according to any one of the preceding claims, wherein said tumour is chosen from a solid tumour; a cutaneous tumour; a mucosal tumour; and a liquid tumour.

6. The formulation for use according to any one of the preceding claims for use by topical administration, preferably comprising, in weight percent with respect to the total weight of the formulation, from 1 to 50%, more preferably from 2 to 20%, even more preferably from 3 to 15% of said at least one ozonized oil.

7. The formulation for use according to claim 6, in a form chosen from oil, gel, and emulsion, preferably oil.

8. The formulation for use according to any one of claims 1 to 5 for use by oral administration, preferably comprising, in weight percent with respect to the total weight of the formulation, from 1 to 5%, more preferably from 2 to 30, of said at least one ozonized oil.

9. The formulation for use according to claim 8, comprising a vehicle compatible with said at least one ozonized oil, preferably in an amount, in weight percent with respect to the total weight of the formulation, from 95 to 99%, more preferably from 97 to 98%.

10. The formulation for use according to claim 8 or 9, wherein said vehicle comprises at least one sweetener and water.

11. The formulation for use according to claim 10, in a form chosen from capsule and drink, preferably a drink, more preferably a syrup.

12. The formulation for use according to any one of claims 1 to 5 for use by administration by injection, preferably consisting of said at least one ozonized oil.

13. The formulation for use according to any one of claims 1 to 5 for use by inhalation administration, preferably wherein said formulation further comprises at least one non-ionic surfactant.

14. The formulation for use according to any one of claims 1 to 13, wherein the treatment of a tumour comprises the administration of said formulation until a U Cor/U Carr ratio is reached between anti-oxidizing species (U Cor) and oxidizing species (U Carr) in the patient's blood comprised between 4 and 12, preferably comprised between 5 and 10, more preferably comprised between 6 and 8.

## Patentansprüche

1. Formulierung, umfassend mindestens ein ozonisiertes Öl zur Verwendung bei der Prävention und/oder Behandlung eines Tumors, vorzugsweise eines bösartigen Tumors, wobei das mindestens eine ozonisierte Öl einen Gehalt an Ozoniden von 500 bis 1500 meq Oz/kg aufweist.

2. Formulierung zur Verwendung nach Anspruch 1, wobei die Formulierung zur Verwendung durch Verabreichung ausgewählt aus oral, topisch, durch Injektion, durch Inhalation und Kombinationen davon ist.

3. Formulierung zur Verwendung nach Anspruch 1 oder 2, wobei das mindestens eine ozonisierte Öl einen Gehalt an Ozoniden von 600 bis 1400 meq Oz/kg, vorzugsweise von 700 bis 1300 meq Oz/kg aufweist.

4. Formulierung zur Verwendung nach einem der vorhergehenden Ansprüche, wobei das ozonisierte Öl ein Pflanzenöl ist, das einem Ozonisierungsprozess unterzogen wurde, vorzugsweise ausgewählt aus: Sonnenblumenöl, Olivenöl, Erdnussöl, Arganöl, Traubenkernöl, Jojobaöl, Sojabohnenöl, Maisöl, Palmöl, Baumwollsamenöl, Rapsöl, Kokosöl, Rizinusöl, Leinöl, Borretschöl, Nachtkerzenöl und Mischungen davon, vorzugsweise Sonnenblumenöl.

5. Formulierung zur Verwendung nach einem der vorhergehenden Ansprüche, wobei der Tumor aus einem soliden Tumor; einem kutanen Tumor; einem Schleimhauttumor; und einem flüssigen Tumor ausgewählt ist.

6. Formulierung zur Verwendung nach einem der vorhergehenden Ansprüche zur Verwendung durch topische Verabreichung, vorzugsweise umfassend, in Gewichtsprozent in Bezug auf das Gesamtgewicht der Formulierung, von 1 bis 50 %, bevorzugter von 2 bis 20 %, noch bevorzugter von 3 bis 15 % des mindestens einen ozonisierten Öls.

7. Formulierung zur Verwendung nach Anspruch 6 in einer Form, ausgewählt aus Öl, Gel und Emulsion, vorzugsweise Öl.

8. Formulierung zur Verwendung nach einem der Ansprüche 1 bis
5 zur Verwendung durch orale Verabreichung, vorzugsweise umfassend, in Gewichtsprozent in Bezug auf das Gesamtgewicht der Formulierung, von 1 bis 5 %, bevorzugter von 2 bis 3 %, des mindestens einen ozonisierten Öls.

9. Formulierung zur Verwendung nach Anspruch 8, umfassend einen
Träger, der mit dem mindestens einen ozonisierten Öl kompatibel ist, vorzugsweise in einer Menge in Gewichtsprozent in Bezug auf das Gesamtgewicht der Formulierung, von 95 bis 99 %, bevorzugter von 97 bis 98 %.

10. Formulierung zur Verwendung nach Anspruch 8 oder 9, wobei der Träger mindestens ein Süßungsmittel und Wasser umfasst.

11. Formulierung zur Verwendung nach Anspruch 10 in einer Form, ausgewählt aus Kapsel und Getränk, vorzugsweise einem Getränk, bevorzugter einem Sirup.

12. Formulierung zur Verwendung nach einem der Ansprüche 1 bis
5 zur Verwendung durch Verabreichung durch Injektion, vorzugsweise bestehend aus dem mindestens einen ozonisierten Öl.

13. Formulierung zur Verwendung nach einem der Ansprüche 1 bis
5 zur Verwendung durch Inhalationsverabreichung, wobei die Formulierung vorzugsweise ferner mindestens ein nichtionisches Tensid umfasst.

14. Formulierung zur Verwendung nach einem der Ansprüche 1 bis 13, wobei die Behandlung eines Tumors die Verabreichung der Formulierung umfasst, bis ein U Cor/U Carr-Verhältnis zwischen antioxidierenden Spezies (U Cor) und oxidierenden Spezies (U Carr) im Blut des Patienten im Bereich zwischen 4 und 12, vorzugsweise im Bereich zwischen 5 und 10, bevorzugter im Bereich zwischen 6 und 8 erreicht wird.

## Revendications

1. Formulation comprenant au moins une huile ozonisée à utiliser dans la prévention et/ou le traitement d'une tumeur, de préférence une tumeur maligne, dans laquelle ladite au moins une huile ozonisée a une teneur en ozonides de 500 à 1500 meq O₂/kg.

2. Formulation à utiliser selon la revendication 1, dans laquelle ladite formulation est destinée à une administration choisie parmi les suivantes : orale, topique, par injection, par inhalation et des combinaisons de celles-ci.

3. Formulation à utiliser selon la revendication 1 ou 2, dans laquelle ladite au moins une huile ozonisée a une teneur en ozonides de 600 à 1400 meq O₂/kg, de préférence de 700 à 1300 meq O₂/kg.

4. Formulation à utiliser selon l'une quelconque des revendications précédentes, dans laquelle ladite huile ozonisée est une huile végétale ayant subi un processus d'ozonisation, de préférence choisie parmi : l'huile de tournesol, l'huile d'olive, l'huile d'arachide, l'huile d'argan, l'huile de pépins de raisin, l'huile de jojoba, l'huile de soja, l'huile de maïs, l'huile de palme, l'huile de coton, l'huile de colza, l'huile de coco, l'huile de ricin, l'huile de lin, l'huile de bourrache, l'huile d'onagre, et leurs mélanges, de préférence l'huile de tournesol.

5. Formulation à utiliser selon l'une quelconque des revendications précédentes, dans laquelle ladite tumeur est choisie parmi une tumeur solide, une tumeur cutanée, une tumeur muqueuse et une tumeur liquide.

6. Formulation à utiliser selon l'une quelconque des revendications précédentes à utiliser par administration topique, comprenant de préférence, en pourcentage en poids par rapport au poids total de la formulation, de 1 à 50 %, plus préférentiellement de 2 à 20 %, encore plus préférentiellement de 3 à 15 % de ladite au moins une huile ozonisée.

7. Formulation à utiliser selon la revendication 6, sous une forme choisie parmi l'huile, le gel et l'émulsion, de préférence l'huile.

8. Formulation à utiliser selon l'une quelconque des revendications 1 à
5 pour une utilisation par administration orale, comprenant de préférence, en pourcentage en poids par rapport au poids total de la formulation, de 1 à 5 %, plus préférentiellement de 2 à 3 %, de ladite au moins une huile ozonisée.

9. Formule à utiliser selon la revendication 8, comprenant un
véhicule compatible avec ladite au moins une huile ozonisée, de préférence dans une quantité, en pourcentage en poids par rapport au poids total de la formulation, de 95 à 99 %, plus préférentiellement de 97 à 98 %.

10. Formulation à utiliser selon la revendication 8 ou 9, dans laquelle ledit véhicule comprend au moins un édulcorant et de l'eau.

11. Formulation à utiliser selon la revendication 10, sous une forme choisie entre capsule et boisson, de préférence une boisson, plus préférentiellement un sirop.

12. Formulation à utiliser selon l'une quelconque des revendications 1 à 5 pour une administration par injection, de préférence constituée de ladite au moins une huile ozonisée.

13. Formulation à utiliser selon l'une quelconque des revendications 1 à 5 pour une administration par inhalation, de préférence dans laquelle ladite formulation comprend en outre au moins un agent tensioactif non ionique.

14. Formulation à utiliser selon l'une quelconque des revendications 1 à 13, dans laquelle le traitement d'une tumeur comprend l'administration de ladite formulation jusqu'à ce que le rapport U Cor/U Carr entre les espèces anti-oxydantes (U Cor) et les espèces oxydantes (U Carr) dans le sang du patient soit compris entre 4 et 12, de préférence compris entre 5 et 10, plus préférentiellement compris entre 6 et 8.
